# EUROPEAN PATENT APPLICATION

(11) **EP 3 804 740 A1**
(43) Date of publication of application: **14.04.2021**
(21) Application number: 19202847.0
(22) Date of filing: 11.10.2019
(51) Int. Cl.: A61K 36/63, A61K 8/00, A61P 31/00

(54) **COMPOSITIONS OF THE OLEA EUROPAEA V. SYLVESTRIS WITH ANTIMICROBIAL EFFICACY**

(71) Applicant: TAPROGGE GmbH, 58300 Wetter (DE); Cuatro Molinos S.A., 11380 Tarifa (ES)
(72) Inventor: Depmer, Ulrich, 11380 Tarifa (ES); Taprogge, Detlef, 58093 Hegen (DE)
(74) Representative: Krauss, Jan

(57) **Abstract**

The invention relates to methods for preparing an olive (*Olea europaea* subsp. *europaea*), preferably acebuche (*Olea europaea subsp. europaea* var. *sylvestris*) composition, the olive or acebuche composition as prepared, and uses thereof in the prevention or treatment of a bacterial or fungal infection in mammals, preferably infection by antibiotic resistant or multi-resistant bacteria. Other uses relate to functional food additives, in particular an animal feed additive, or to uses in cosmetics

## Description

The invention relates to methods for preparing an olive (*Olea europaea* subsp. *europaea*), preferably acebuche (*Olea europaea subsp. europaea* var. *sylvestris*) composition, the olive or acebuche composition as prepared, and uses thereof in the prevention or treatment of a bacterial or fungal infection in mammals, preferably infection by antibiotic resistant or multi-resistant bacteria. Other uses relate to functional food additives, in particular an animal feed additive, or to uses in cosmetics.

### FIELD OF THE INVENTION

The olive tree (*Olea europaea L*.) is an ancient cultivated and character plant of the family *Oleaceae.* It includes 30 genera and 600 different species. Genus Olea include 50 species that can be found in various continents and regions, including Africa, China, India, the USA and Australia (Iming, 2005). *Olea europaea* is divided into different subspecies: among others *Olea europaea* subsp. *europaea*, the European olive tree, and *Olea europaea* subsp. *africana*, which grows in Africa, Madagascar, India and China. Of both species there exist the wild and cultured forms (Iming, 2005). Olive trees can grow up to 1000 years old and require very little water, which explains their occurrence in dry areas (Iming, 2005). Most of the world olive harvest comes from the Mediterranean (Lieberei, 2007).

The wild olive, *Olea europaea var. sylvestris*, the ancestor of all olive plants, is generally known in Spain under the name "acebuche". The distribution of acebuche trees on, for example, the Spanish peninsula is irregular; they grow spontaneously and occasionally as isolated bushes or groups of bushes, predominantly in the Mediterranean.

Leaves of the olive tree are quite different from those of fruit trees, and leaves are sometimes used as a natural remedy. Olive trees are furthermore very resistant to fungal and bacterial attacks. These antibiotic and protective properties seem to be based on numerous active compounds as produced by the olive, such as oleuropein which is currently one of the most studied (Iming, 2005).

The active compounds of the olive tree are present in leaf, fruit, bud, stem, branch and root in different concentrations (Effect, 2017). It is speculated that olive leaves can have immune-enhancing, anti-inflammatory and blood pressure-reducing effects (Iming, 2005). Nevertheless, little is known about the actual medical effects involved.

A large number of bioactive substances have been characterized in the olive tree, and can be assigned to the substance classes of, for example, secoiridoid glycosides, phenolic compounds, flavonoids, monoterpenes, triterpenes, steroids, quinoline alkaloids, carotenoids, chlorophylls, phenolic carboxylic acids, tannins, and vitamins (Effect, 2017).

Olive tree leaves accumulate during the harvesting of olives and thus provide an easily accessible by-product that can be used for other purposes (Cayuela, 2006). The development of new uses for by-products of olive oil production is of great importance, especially for the olive groves. Research has focused increasingly on the chemical composition of the olive fruits, and little is known about the chemical components of olive leaves, especially the wild olive tree acebuche.

Since their discovery over 70 years ago, antibiotics have been our main weapon in the treatment of bacterial infections, including life-threatening hospital-acquired infections. However, antibiotics are often routinely prescribed and taken, often inappropriately. Antibiotics are also used in livestock farming for treatment, disease prevention and growth promotion. Antibiotics are also found in the environment, for example in some water supply systems. (WHO, 2018).

Antibiotic resistance is an expected natural mechanism that occurs where an antibiotic that would normally stop the growth of a particular bacterial species no longer has any effect (Krämer, 2007). Worldwide, around 700,000 people die each year from antimicrobial resistance (AMR). In the EU there are about 25,000 people. It is feared that antimicrobial resistances could cause more deaths than cancer by year 2050 (Parliament, 2018).

Infections with resistant bacteria are often difficult to cure, sometimes even incurable, and their numbers are rising. However, research into new, effective antibiotics is very expensive and time-consuming, and often antibiotic resistance emerge even after the market launch of a new antibiotic. At present, only very few new antibiotics are in development. If no effective new antibiotics are found and the resistances continue to spread, society is threatened with a return to circumstances such as those in which before the discovery of the antibiotics, when children were often involved in a simple pneumonia died and doctors for meningitis were powerless. Also, a number of complicated medical interventions and diagnostics would be made impossible, if effective antibiotics were no longer available for prophylaxis.

The emergence of resistant bacteria is a serious problem in health care facilities, leading to life-threatening blood and wound infections and pneumonia, among other things. Antibiotic resistance leads to an increase in treatment costs due to longer hospital stays, higher expenditure on antibiotics and treatment, and indirect costs to family and society. In many countries in the European Region, antibiotics do not require a prescription. Data on antibioticresistant infections are often not collected, so that the extent of the problem is not documented, despite a high level of awareness among physicians. Nosocomial infections are among the most common infections in Germany. The problem of antibiotic resistance and its spread, which is precisely associated with these infections, represents one of the greatest challenges for modern medicine (Braun AG, 2018).

Ahmed et al. (in: Ahmed, Ali & S. Rabii, Nancy & Garbaj, Aboubaker & Abolghait, Said. (2014). Antibacterial effect of olive (Olea europaea L.) leaves extract in raw peeled undeveined shrimp (Penaeus semisulcatus). International Journal of Veterinary Science and Medicine. 2. 10.1016/j.ijvsm.2014.04.002) evaluated the effect of olive leaves extract from *Olea europaea L.* on the microbial load of raw peeled undeveined shrimp and discussed the potential use of olive leaves extract formulations to improve the microbial quality and as a natural preservative.

Paudel et al. (in: Paudel, Shambhu & Magrati, Thakur & Lamichhane, Jay Ram. (2011). Antimicrobial activity of wild olive crude extracts in vitro. International Journal of Pharma Sciences and Research) disclose a screening of wild olive crude extracts for antibacterial activity against five different bacterial human pathogens, wherein the extract obtained with methanol appeared to be the most effective against all pathogenic bacteria.

DE102011108948A1 describes a clear, liquid formulation for lipophilic substances that are very difficult to dissolve in water, which contains exactly one solubilizer and can only be produced by stirring, without further complex process steps.

In further studies (e.g. in: Korukluoglu, Mihriban & Sahan, Yasemin & YIGIT, AYCAN & Tümay Özer, Elif & Gucer, Seref. (2010). Antibacterial activity and chemical constitutions of Olea europaea L. leaf extracts. Journal of Food Processing and Preservation. 34. 383 - 396. 10-1111/j.1745-4549.2008.00318.x), an olive leaf extract inhibited the growth of a number of bacteria and moulds such as *Escherichia Coli, Klebsiella pneumoniae, Bacillus cereus, Aspergillus flavus, Aspergillus parasiticus.* The aqueous extract of olive leaves had no antibacterial effect against the test microorganisms, whereas acetone extract showed inhibitory effect on *Salmonella enteritidis, Bacillus cereus, Klebsiella pneumoniae, Escherichia coli, Enterococcus faecalis, Streptococcus thermophilus* and *Lactobacillus bulgaricus.* Furthermore, the antimicrobial activities of some phenolic compounds against microorganisms were tested. The most effective compound was found to be oleuropein while syringic acid was found ineffective. Experiments of this kind have not yet been carried out with the wild olive "acebuche".

Wang et al. (in: Wang, Xiao-Fei & Li, Chen & Shi, Yan-Ping & Di, Duo-Long. (2009). Two new secoiridoid glycosides from the leaves of Olea europaea L. Journal of Asian natural products research. 11. 940-4. 10.1080/10286020903310979) disclose two secoiridoid glycosides, oleuricines A (1) and B (2), together with five known triterpenoids, beta-amyrin, oleanolic acid, erythrodiol, urs-2beta,3beta-dihydroxy-12-en-28-oic acid, and beta-maslinic acid, isolated from the EtOAc-soluble part of EtOH extract of the leaves of Olea europaea L. The structures of these compounds were elucidated by various spectroscopic methods, including intensive 1D, 2D NMR, and HR-ESI-MS techniques.

In view of the above it is an object of the present invention to provide new and effective antibacterial compounds and compositions derived from olive or acebuche. Other objects and advantages of the present invention will become apparent for the person of skill upon studying the following more detailed description of the invention.

In a first aspect thereof the present invention solves the above object by providing a method for preparing an olive (Olea europaea subsp. europaea), preferably acebuche (Olea europaea subsp. europaea var. sylvestris) composition, comprising the steps of a) providing parts of an olive or acebuche plant; and b) removing moisture from said olive or acebuche parts by dewatering or drying of said olive or acebuche parts; and c) comminution of said olive or acebuche parts of step b) by chopping, grinding, milling, pulverizing or other methods of comminution of said olive or acebuche parts to obtain a pulverized olive or acebuche; d) suitably sterilizing said pulverized olive or acebuche, preferably at a temperature of 121°C or 134°C to obtain a sterilized pulverized olive or acebuche; and, as may be desired, e) blending of said sterilized pulverized olive or acebuche with water and a thickening agent to obtain an antibiotically or antifungally active olive or acebuche gel; or, as may be desired; or f) extracting an antibiotically or antifungally active agent from the pulverized olive or acebuche from step c) by extraction, preferably CO₂ extraction; suspending of said active agent in a non-toxic solubilizer and emulsifying agent to obtain an antibiotically or antifungally active olive or acebuche suspension.

It was surprisingly found that the pharmacological differences are particularly prominent between the cultured olive vs. acebuche (see for example table 2, below).

In a second aspect thereof the present invention solves the above object by providing an antibiotically or antifungally active sterilized pulverized olive or acebuche, obtained from steps a, b), c), or d) of the method according to the present invention as above.

In a third aspect thereof the present invention solves the above object by providing an olive or acebuche composition, produced by a method according to the present invention, optionally comprising pharmaceutically or cosmetically acceptable carriers, diluents or excipients.

In a fourth aspect thereof the present invention solves the above object by providing a sterilized pulverized olive or acebuche as described herein or the olive or acebuche composition as described herein for use in the prevention or treatment of a bacterial or fungal infection in mammals, preferably for use in the prevention or treatment of infection by antibiotic resistant or multi-resistant bacteria.

In a fifth aspect thereof the present invention solves the above object by providing the use of the sterilized pulverized olive or acebuche as described herein or the olive or acebuche composition as described herein as a functional food additive, in particular an animal feed additive.

In a sixth aspect thereof the present invention solves the above object by providing a use of the sterilized pulverized olive or acebuche as described herein the olive or acebuche composition as described herein as a cosmetic product and/or in cosmetic methods.

### DETAILED DESCRIPTION OF THE INVENTION

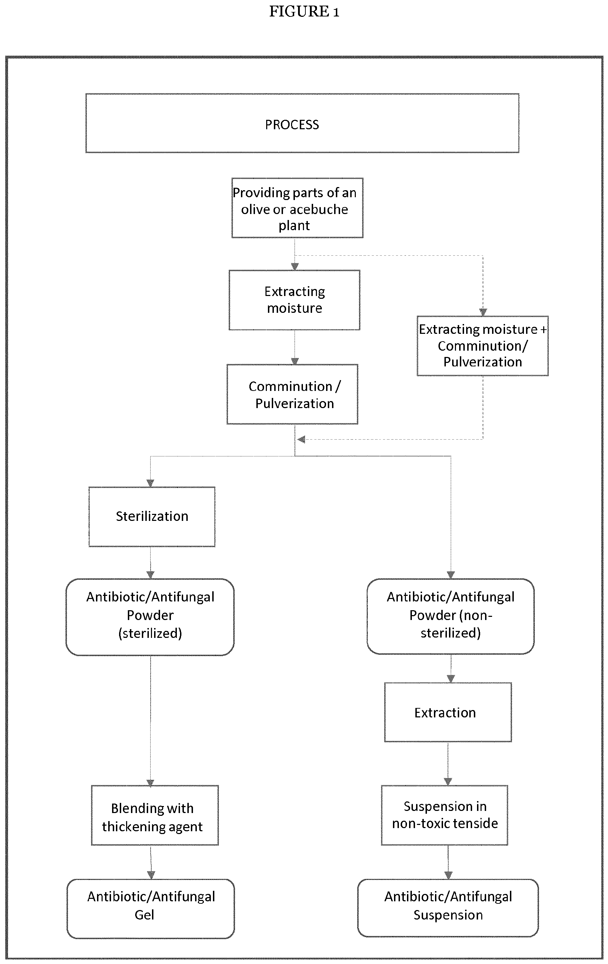

As explained above, in a first aspect of the invention, the above problem is solved by providing a method for preparing an olive (*Olea europaea* subsp. *europaea*), preferably acebuche (*Olea europaea* subsp. *europaea* var. *sylvestris*) composition, comprising the steps of a) providing parts of an olive or acebuche plant; and b) removing moisture from said olive or acebuche parts by dewatering and/or drying of said olive or acebuche parts; and c) comminution of said olive or acebuche parts of step b) by chopping, grinding, milling, pulverizing and/or other suitable methods of comminution of said olive or acebuche parts in order to obtain a pulverized olive or acebuche; d) suitably sterilizing said pulverized olive or acebuche, preferably at a temperature of about 120°C to about 140°C, preferably at about 121°C or about 134°C to obtain a sterilized pulverized olive or acebuche; and, optionally, e) blending of said sterilized pulverized olive or acebuche with water and a thickening agent in order to obtain an antibiotically or antifungally active olive or acebuche gel; or f) extracting an antibiotically or antifungally active agent from the pulverized olive or acebuche from step c) by extraction, preferably CO₂ extraction; suspending of said active agent in at least one non-toxic solubilizer and/or emulsifying agent in order to obtain an antibiotically or antifungally active olive or acebuche suspension (see Fig. 1).

Therefore, the present invention relates to two particularly important compositions having desirable properties, a) an antibiotically or antifungally active olive or acebuche gel; and b) an antibiotically or antifungally active olive or acebuche suspension in at least one non-toxic solubilizer and/or emulsifying agent.

The best researched secoiridoid glycoside of the olive plant is oleuropein. Oleuropein is present in all parts of the olive plant, and various beneficial effects are attributed to this compound, including antibacterial, antiviral, anti-inflammatory, antirheumatic, antioxidant or cardioprotective activity (Fleming, 1973). Olive tree leaves contain between 60 - 90 mg/g dry mass oleuropein (Khan, 2007).

In addition to oleuropein, the olive leaf contains other phenolic components such as dimethyloleuropein, ligstrosides, verbacosides, oleoside dimethyl ester and oleurosid (Cayuela, 2006). Olive leaves also contain flavonoids. These include rutin, luteolin and apigenin. Flavonoids reduce oxidative damage by absorbing UV light and prevent oxidation by free radicals (Cayuela, 2006).

Guinda et al. describes that the olive tree is the suitable raw material for the production of oleanolic acid and other pentacyclic triterpenes (Guinda, 2010). These include ursolic acid, betulinic acid, maslic acid, erythrodiol and uvaol. Guinda et al. already describe that the content of pentacyclic triterpenes in the olive leaf is higher than in the fruit itself. Oleanolic and ursolic acids are most strongly represented (Guinda, 2010, Bianchi, 1992). The concentration of the various triterpenes is strongly dependent on the stage of development of the fruit and plant as well as the cultivar (Guinda, 2010, Stiti, 2007).

Oleanol and maslic acid are desirable raw materials for the pharmaceutical and cosmetics industries that can be extracted from olive leaves (Guinda, 2010). For this reason, the leaves of the wild olive "acebuche" were examined for their pentacyclic triterpene profile. These could possibly have an influence on the antimicrobial efficacy.

In the context of the present invention, the inventors developed a method for optimal processing of an olive or acebuche composition with antimicrobial efficacy. One particular advantage of the method according to the present invention is that two decisive steps, namely extracting moisture and comminution, are carried out in a single device, which considerably reduces the investment and saves operating and energy costs.

Furthermore, it was found that in the context of the method, the energy consumption required is significantly lower when a Venturi nozzle is used.

Most importantly, it was also surprisingly found that the olive or acebuche parts processed by the Venturi device result in pulverized olive or acebuche with a significantly higher antibiotic and antifungal effect than the material processed in conventional mills, i.e. without a Venturi device. It was also found that the pulverized olive or acebuche as produced with said Venturi device has a particularly long shelf life.

The following table 1 shows the decrease in CFU of bacterium *Pseudomonas aeruginosa* when treated with olive substrates processed by conventional milling methods compared to olive substrates processed by the Venturi device.

| Incubation time in min. | CFU; treated with olive substrate processed with hammer mills | CFU; treated with olive substrate processed with Venturi device |
|---|---|---|
| 0 | 490,000 | 420,000 |
| 3 | 420,000 | 3,500 |
| 6 | 490,000 | 11,000 |
| 9 | 470,000 | 810 |
| 12 | 300,000 | 300 |
| 30 | 99,000 | 1 |
| 60 | 4,400 | 1 |

Olive leaf extracts as natural antibiotic or antifungal agents with a broad spectrum of antimicrobial activities are a groundbreaking step in the battle against infection, in particular in case of (multiple) antibiotic resistant - and thus particularly problematic - microbes.

The active sterilized pulverized olive or acebuche, or the olive or acebuche composition, for example, serves as a basis for interactive wound dressings, but can also be used for antiseptic, topical body disinfection, medical washing at predilection sites of germ colonisation and in particular for the reduction and/or elimination of the dominant germs in wound treatment and their multi-resistant variants.

The term "providing" in the context of the present invention means the preparation of olive and acebuche plants as feed material for the method described herein. It may be necessary to clean said olive and acebuche plants from surface impurities as present, e.g. by water bath, rinsing or spraying or further methods known by a skilled person. It may also be necessary to bring the olive and acebuche plants to a size that can be considered suitable for handling the olive and acebuche plants. Respective methods are also known to the person of skill.

"Dewatering" or "drying" as used herein usually and preferably relates to a direct drying, wherein, for example, said direct drying is achieved by simple exposure of the feed material to air (also called natural air drying), or heated air of enhanced air flow drying, by means of, for example, ventilators, blowers or other devices known in the prior art; or indirect drying, wherein indirect drying is achieved by contact drying or drum drying; or dielectric drying, by means of, for example by microwave; or infrared drying; vacuum drying; or freeze drying, wherein these methods are may suitable to reduce drying time or to allow for a higher degree of sensitivity as may be required. Methods to shorten the time of moisture extraction using elevated temperatures have limitations, as too elevated temperatures and too long duration of elevated temperature exposure may lead to deterioration or destruction of the antibacterially/antifungally active components of the raw material. For the avoidance of any fouling or decaying processes from handling and storing of the dried material, the material is preferably dried to a degree of 95 % dry substance (DS) or higher. The degree of moisture content may be optimized to such lower or higher %-age of dry substance as may be recommendable for the subsequent comminution and pulverization step.

"Comminution" as used herein is usually and preferably carried out in one or more steps in order to achieve the desired degree of pulverization. The most desired degree of pulverization is determined considering the cost of comminution on the one hand, and the effectiveness of antibacterial or antifungal impact of the resulting pulverized material on the other hand. In accordance to the herein described invention, desired particle size of olive or acebuche parts ranges between 1 µm and 1000 µm, more preferably 40 µm and 500 µm, most preferably between 125 and 250 µm. The desired degree of pulverization is achieved by processing the olive or acebuche parts several times in a comminution device or by using different types of comminution devices which allow the handling of preferred particle sizes. Typical comminution devices are selected from crushers, shredders, choppers, cutters, grinders and mills, e.g. hammer mills, ball mills, impact mills, rotary mills, centrifugal mills, jet mills, bark mills, planetary mills, mortars, pulverizer, micronizers, ultrasonic micro-mills or micro-grinders and the like, but without being limited to it. Depending on specification requirements classification of the pulverized material may be added to this step to separate the plant material into the required size classes. This may also be useful to pulverize particles which have been classified as too large again to reach the desired size class.

As used herein, the term "sterilization" means the removal of the natural contamination of the pulverized olive/acebuche, for example, with aerobic spore formers using various sterilization processes. The different types of sterilization can be selected from thermal sterilization, in which the micro-organisms are killed by heat, for example by heating in a moist state (steam sterilization), or heating in a dry state (hot air sterilization), or fractionated sterilization, in which heating is repeated successively, or by physical sterilization techniques, or other sterilization methods known in the prior art. In the context of the present disclosure sterilization at 121°C and 134°C in a vacuum sterilizer is preferred, wherein the temperature is suitable to preserve the pharmaceutically active ingredients, wherein optionally sterilization is carried out by steam sterilization.

As used herein the term "blending" means mixing or homogenizing of the sterilized pulverized olive or acebuche with water for injection and a thickening agent, as described herein below, to obtain an antibiotically or antifungally active olive or acebuche gel, and wherein it is mixed until a uniform gel is formed. There is a variety of methods known to the skilled person that can be used to achieve the required result.

"Extracting" as used herein describes the isolation of an antibiotically or antifungally active agent from the pulverized olive or acebuche obtained by the herein described method. According to the present invention, the extraction is preferably achieved by CO2 extraction. The extraction can be carried out in batch mode, as the extractor can only be emptied and refilled at atmospheric pressure. During extraction the supercritical carbon dioxide (CO2) percolates at high pressure through the raw material and extracts the soluble substances from the pulverized olive or acebuche (eq. extract). The dissolved substances are divided into fractions with different compositions, by gradually reducing the pressure. In the first fraction (separator 1) the less soluble substances accumulate, whereas the more soluble substances are collected in the following separator 2. Further types of solid-liquid extractions can be selected, such as maceration and decantation, without being limited to these, as long as the active agent remains fully effective.

In a preferred embodiment of the method according to the present invention, steps b) and c) are performed simultaneously by subjecting said olive or acebuche parts to forces generated by dynamic airflow, preferably by passing said olive or acebuche together with air or other suitable gas, preferably pre-heated air, through a Venturi nozzle. The granted patents US 7,429,008 B2, US 7,500,830 B2 and US 7,909,577 B2 disclose the pulverization of a material that continues to be subject to moisture extraction and drying during pulverization by means of an airflow generator coupled to a Venturi nozzle, but using, for example, polymers or waste in general as feed material. Further, the patent application WO 2013/052583 A2 generally discloses dewatering, pulverizing, and pyrolysis of biomasses, such as sewage sludge. The patent application WO 2013/075003 A1 provides a method for the production of eggshell powder for use in the manufacture of bio-based products. The method involves a high air velocity at room temperature to pulverize the egg shell and separate the egg shell component from the inner membrane component. Nevertheless, it has not yet been possible to develop a method with which it is possible to simultaneously dewater and dry parts of the olive or acebuche and comminute them simultaneously as it is provided by the present invention. The active agents of the olive or acebuche are preserved and the powder has a long shelf life.

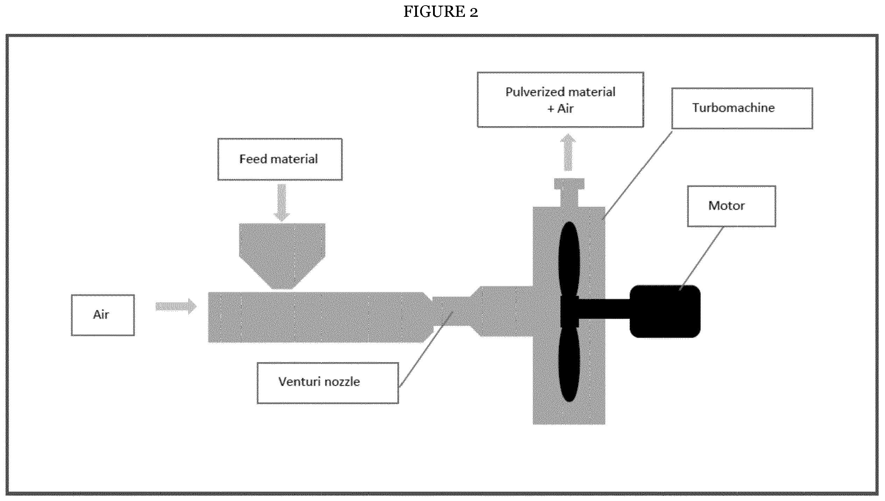

Thus, the olive and/or acebuche parts are exposed to dynamic air flow, e.g. by passing said olive or acebuche parts together with air through said Venturi nozzle. In doing so, the airflow draws the feed material through the Venturi nozzle arranged in connection with the device. The movement of the air through the Venturi nozzle then accelerates both the air and the olive or acebuche contained in the airflow. The airflow is, for example, generated by rotation of blades in a turbomachine or by another suitable device for drawing airflow. The airflow and the dried and pulverized olive or acebuche leave the device via outlet nozzle provided by device, wherein the pulverized olive or acebuche may be separated from the airflow by sieve, cyclone or similar methods know by a person skilled in the art. Simultaneously with comminution, the airflow is heated by energy losses (heat) from the operation of the turbomachine. Then, the accelerated airflow (and the pulverized olive or acebuche) absorbs this heat and increases its temperature. Physically, air at higher temperature is able to absorb relatively more moisture than air at lower temperature. Due to this natural phenomenon, comminution and pulverization and the utilization of heat losses complement each other in the Venturi device principles. Comminution of the feed material pulled through the device including Venturi facilitates enlargement of the feed material's surface for the transfer of moisture to the airflow, and the increased air temperature from the operation of the turbomachine allows an increased moisture transfer from the material to the airflow. The Venturi device can be used to completely or partially implement steps b) and c) of the method according to the present invention. Preferably, the feed point of the olive or acebuche parts may be in front of the inlet pipe in conjunction with the device (see Fig. 2).

In a further preferred embodiment of the method according to the present invention, the thickening agent as used is selected from a gelling agent, such as gellan gum, alginic acid, sodium alginate, potassium alginate, ammonium alginate, calcium alginate, propylene glycol alginate, agar-agar, carrageenan, furcellaran, locust bean gum, guar gum, traganth, gum arabic, xanthan gum, karaya, tara gum, pectin, cellulose, gelatin, and modified starch.

In yet another preferred embodiment of the method according to the present invention, said non-toxic solubilizer and/or emulsifying agent is selected from a tenside, wherein the tenside is preferably selected from Kolliphor®, most preferably Kolliphor® RH40. Further tensides can be selected from Nonoxinol-9, Octoxinol-9 (Triton X-100®), Polysorbat 20 (Tween 20®), Octyl-β-Glucosid, and N-Octyl-β-D-1-thioglucopyranosid. Other non-toxic solubilizers and/or emulsifying agents can be selected from ethanol, acetone, hexane, chloroform, and *Methyl tert-butyl ether (MTBE).*

The olive or acebuche parts for the method according to the invention can be selected from leaves, twigs, root bark, stem bark, fruit or oil production residue and combinations thereof. As used herein the term "parts" shall refer to all suitable constituents of the olive and acebuche plants, e.g. in form of leaves, twigs, bark of stems or roots, olives or products and residue from the processing of olives. Parts can for example also refer to the plant growing in the soil, harvested parts of the plant, discarded parts of the plant, already processed parts of the plant and dead parts of the plant.

Preferred is a method according to the present invention wherein at least one active agent is contained in the composition as prepared that is selected from antibiotically or antifungally compounds. Preferred agents are selected from the class of iridoids or phenols, and preferably at least one organic compound selected from the group of oleuporein, oleacein, oleocanthal, tyrosol, and hydroxytyrosol. Furthermore, in the context of the present disclosure, the active agent can further comprise oleuroside, ligstroside, verbascoside, flavonoide, maslinic acid, apigenin, luteolin, and oleanolic acid. Most preferred are oleuporein, oleacein, oleocanthal, tyrosol, and hydroxytyrosol.

In a second aspect thereof, the present invention solves the above mentioned problem by providing an antibiotically or antifungally active sterilized pulverized olive or acebuche product/composition, obtained by a method according to the present invention.

Particularly preferred is an antibiotically or antifungally active sterilized pulverized olive or acebuche according to the present invention that is obtained from steps a, b), c), and d) of the method according to the present invention.

The term "antibiotically active" as used herein generally refers to the activity to act against infections caused by microorganisms. These microorganisms are primarily bacteria, but they also comprise for example protozoa. The effect can comprise killing the microorganisms, and/or inhibiting growth and/or reproduction. In the context of the present invention, the antibacterial agent is particularly active and effective against microorganisms that are resistant to commercial antibiotics, and especially against multi-resistant microorganisms (i.e. bacteria or fungi that are resistant against at least two antibiotics, such as commercial antibiotic substances). "Antifungally active" as used herein, describes the activity to act against an infection caused by fungi, such as molds. The effect may comprise killing the fungi or inhibiting growth or proliferation. The term "resistant" as used herein refers to the properties of microorganisms that enable them to attenuate or completely neutralise the action of commercial antibiotic substances. The term "multi-resistant" as used herein refers to the property of microorganisms to attenuate or completely neutralise the action of at least two different commercial antibiotic substances.

Preferred is the antibiotically or antifungally active sterilized pulverized acebuche according to the present invention, wherein said pulverized olive or acebuche is a powder and consists of more than 95% by weight of dry biomass. The term "dry biomass" as used herein describes the actual dried amount of substance mass without water, or other constituents, such as other liquids. "% by weight" as used herein, means the mass proportion of a mixture, in particular of the pulverized olive or acebuche. The composition of the pulverized olive or acebuche depends on the proportion of a single component in 100 grams of the mixture. In the context of the present invention, it is preferred that more than about 95% of 100 g is made up of dried biomass.

In a third aspect thereof, the present invention solves the abovementioned problem by providing an olive or acebuche composition, produced by a method according to the present invention. Optionally and preferably said composition comprises pharmaceutically or cosmetically acceptable carriers, diluents or excipients.

In another preferred aspect, the invention relates to an antibacterial or antifungal composition comprising at least one organic compound selected from oleacein, hydroxytyrosol and oleocanthal. The composition preferably is a pharmaceutical or cosmetic composition as described herein.

In another preferred aspect, the invention relates to a composition as above for use in the prevention or treatment of a bacterial or fungal infection in mammals, preferably for use against infection by antibiotic resistant or multi-resistant bacteria

A "pharmaceutically acceptable carrier, diluent or excipient" as used herein refers to an ingredient in a pharmaceutical formulation or composition, other than an active ingredient, which is nontoxic to a subject. Pharmaceutically acceptable carriers, diluents or excipients include any and all suitable solvents, dispersion media, coatings, further antibacterial and antifungal agents, isotonic and absorption delaying agents that are physiologically compatible. The carriers include various preservatives, antibacterial and antifungal agents such as parabens, chlorobutanol, phenol, sorbic acid and the like. It may also be desirable to include isotonic agents such as sugar, sodium chloride and the like in the inventive compositions. In addition, prolonged absorption of the injectable dosage form can be achieved by using absorption retardants such as aluminium monostearate and gelatin.

Regardless of the route of administration selected, the compositions of the present invention, which may be used in a suitable hydrated form, and/or the pharmaceutical compositions of the present invention, are formulated into pharmaceutically acceptable dosage forms by conventional methods known to those of skilled in the art. Actual dosage levels of the active ingredients in the pharmaceutical compositions of the present invention may be varied. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The composition must be sterile and fluid to the extent that the composition is deliverable by syringe. In many cases, isotonic agents, for example, sugars, polyalcohols such as mannitol or sorbitol, and sodium chloride are included in the composition.

The compositions of the invention may be administered locally or systemically. Administration will generally be parenterally, e.g., intravenously. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, antioxidants, chelating agents, and inert gases and the like.

A "cosmetically acceptable carrier, diluent or excipient" as used herein refers to a suitable ingredient in a cosmetic formulation other than an active ingredient. Usually, such an ingredient has to be suitable for use in topical contact with tissues, for example the skin, without undue toxicity, incompatibility, instability, irritation, allergic response, or the like. Cosmetically acceptable carriers, diluents or excipients can for example include water, liquid or solid emollients, solvents, humectants, thickeners and powders facilitate the distribution of the composition when it is applied for example to the skin, hair and/or nails.

A further particularly preferred embodiment of the invention is an olive or acebuche composition according to the present invention, wherein said composition is a gel, preferably a homogenous colloidal gel. In the context of the invention, "colloidal gel" means those in which the lipophilic pulverized olive or acebuche, obtained by the method according to the present invention, is present in loads of up to 100% in particles of colloidal size, i.e. between 1 and 1000 µm, so that the solubility of the hardly soluble lipophilic pulverized olive or acebuche is given and homogeneous distribution of the pulverized olive or acebuche is guaranteed.

Other particularly preferred embodiments relate to the olive or acebuche suspension according to the present invention, wherein said olive or acebuche suspension is an aqueous suspension. As used herein the term particularly comprises the olive or acebuche suspension as water soluble through the use of tensides and respective aqueous solutions as advantageously made available through said use.

Further preferred is the olive or acebuche composition according to the present invention, wherein said composition is a suspension, and wherein the amount of the non-toxic solubilizer, in particular the tenside, is less than 10%, preferably less than 5%, and more preferably less than 3% of the total volume of the olive or acebuche suspension. As mentioned above, the tenside is preferably selected from Kolliphor®, most preferably Kolliphor® RH40.

In another preferred embodiment of the present invention the composition as described herein is in the form of a salve, a lotion, a cream, spray, a gel, a liquid, drops, a capsule or a suppository. Said composition can be administered systemically, i.e. by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. For transmucosal administration the compositions according to the present invention are formulated into liquids, drops, capsules or suppositories. For topical (transdermal) administration, said composition are formulated into salves, lotions, creams, sprays, or gels, as is generally known in the art.

In a fourth aspect thereof, the present invention solves the abovementioned problem by providing the sterilized pulverized olive or acebuche as described herein, or an olive or acebuche composition as described herein, for use in medicine. More preferred is the sterilized pulverized olive or acebuche as described herein, or an olive or acebuche composition as described herein for use in the prevention or treatment of a microbial, such as a bacterial or fungal infection in a mammal, preferably for use against infection by antibiotic resistant or even multi-resistant bacteria. Another aspect relates to the use of the sterilized pulverized olive or acebuche as described herein, or an olive or acebuche composition as described herein for the production of a medicament for the prevention or treatment of a microbial, such as a bacterial or fungal infection in a mammal, preferably for use against infection by antibiotic resistant or even multi-resistant bacteria.

A "mammal", as used herein, may be a farmed animal (e.g. a horse, cow, sheep or pig), a pet (e.g. a cat, dog, rabbit or guinea pig), a rodent or in particular a human. Hence, compositions according to the present invention may be used to treat any of these mammals.

As used herein, the term "treatment" comprises the administration of said composition to said mammal, preferably in a therapeutically effective amount to alleviate the disease or progression of the disease. Therefore, an effective amount is an amount of the composition or the pharmaceutical compositions described herein above that normalize the infection state in the mammal. The amount alleviates symptoms as found for the infection and/or condition, without being toxic to the subject. The dosage regimen will be determined by the attending physician and clinical factors. As is well known in the medical arts, dosages for any one mammal depend upon many factors, including the mammal's size, body surface area, age, the particular compound to be administered, sex, time and route of administration, general health, and other drugs being administered concurrently.

As used herein, the term "prevention" comprises the administration of said composition to said mammal, preferably in a preventively effective amount to refer to reduce, no matter how slight, of a subject's predisposition or risk for developing an infection by antibiotic resistant or multi-resistant bacteria. For prevention, the mammal is preferably a mammal that is at risk or susceptible to the development of an infection by antibiotic resistant or multi-resistant bacteria, wherein the composition is preferably administered by injection. Further, enteral and topical administration can be included in the context of the present disclosure and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrastern injection and infusion.

In yet another preferred embodiment of the present invention, the sterilized pulverized olive or acebuche according to the present invention or the olive or acebuche composition according to the present invention is for use in the prevention or treatment of a bacterial or fungal infection in mammals, preferably infection by antibiotic resistant or multi-resistant bacteria, wherein said bacteria are selected from *Escherichia coli, Staphylococcus aureus, Pseudomonas aeruginosa, Klebsiella pneumoniae, Enterococcus hirae, Enterococcus faecalis, Enterobacter, Mycobacterium tuberculosis, Serratia, Proteus, Providencia, Morganella, Enterococcus faecium, Heliocobacter pylori, Campylobacter, Salmonella, Neisseria gonorrhoeae, Streptococcus pneumoniae, Haemophilus influenza, Shigella Acinetobacter baumannii,* and resistant and multi-resistant strains thereof, and for use in the prevention or treatment of a fungal infection in mammals, wherein said fungi are selected from *Candida albicans* and *Aspergillus brasiliensis.*

In a fifth aspect thereof, the present invention solves the abovementioned problem by providing a functional food additive comprising the sterilized pulverized olive or acebuche, as described herein, or the olive or acebuche composition, as described herein. The abovementioned problem is furthermore solved by providing the use of the sterilized pulverized olive or acebuche, as described herein, or the olive or acebuche composition, as described herein, as a functional food additive, in particular an animal feed additive. As used herein, the term "animal feed additive" means the addition of the composition in feed for farm animals.

Furthermore, as used herein, the term "functional food additive" describes the use of the composition to increase the supply of the active ingredients of the composition to a mammal by supplementing a general diet. The composition may also be taken as a dietary or food supplement in the form of a concentrate or in doses, in particular in the form of capsules, pastilles, tablets, pills, effervescent tablets and other similar forms, powder sachets, liquid ampoules, dropper bottles and similar forms of liquids and powders for ingestion in appropriate small quantities in addition to the general nutrition. In particular, the term "animal feed additive" means the addition of composition to feed for farmed animals, whereby feed for other animal species is not excluded.

In a sixth aspect thereof, the present invention solves the abovementioned problem by providing a cosmetic product comprising the sterilized pulverized olive or acebuche according to the present invention, the olive or acebuche gel or suspension according to the present invention, or the pharmaceutical composition according to the present invention. Another aspect then relates to the use of said sterilized pulverized olive or acebuche, as described herein, the olive or acebuche composition, as described herein, in cosmetics or in a cosmetic method.

As used herein, the term "cosmetics" covers all areas of body care or all areas that serve to maintain, restore, or improve the external appearance of a mammal. In particular, the fields of application of cleaning, care and protection, as well as in particular tooth and mouth care are included within the scope of the present disclosure.

In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than a man-made or engineering technical effect. Where an indefinite or definite article is used when referring to a singular noun, e.g. "a", "an" or "the", this includes a plural of that noun unless something else is specifically stated.

The present invention will now be further described in the following examples, nevertheless, without wanting to be limited thereto. For the purpose of the present invention, all references, patents, and publications cited herein are hereby incorporated by reference in their entirety.

In the Figures,
**Figure 1** shows the schematic method steps for the preparation of an antibiotic/antifungal gel according to the invention or for the preparation of an antibiotic/antifungal suspension according to the invention, including respective intermediate products.
**Figure 2** shows the principle of the device used for the method steps of removing moisture from olive or acebuche parts through dewatering or drying; and the comminution of olive or acebuche parts by chopping, grinding, milling, pulverizing in order to obtain a pulverized olive or acebuche. The steps are performed simultaneously by subjecting the olive or acebuche parts to forces generated by dynamic airflow, preferably by passing said olive or acebuche together with air, preferably pre-heated air, through a Venturi nozzle.
**Figure 3** shows a comparison of active substances (iridoids) of different olive cultivars.
**Figure 4** also shows a comparison of active substances (flavinoids) of different olive cultivars.
**Figure 5** shows another comparison of active substances (terpenes) of different olive cultivars.
**Figure 6** shows yet another comparison of active substances (tyrosol and hydroxytyrosol) of different olive cultivars.

### EXAMPLES

### Example 1: Extraction of olive tree components with supercritical carbon dioxide

The dried and pre-grinded olive tree components are presented to the extractor. The extractions are always carried out in batch mode, as the extractor can only be emptied and refilled at atmospheric pressure. During the extraction, the supercritical carbon dioxide (CO₂) percolates at high pressure through the raw material and extracts the soluble substances from the raw material (i.e. extract). The dissolved substances can be divided into fractions with different compositions, by gradually reducing the pressure. In the first fraction (separator 1) the pressure can be the more difficult soluble substances accumulate, whereby in the following separator 2 the more easily soluble substances can be collected.

An extraction variant with only one separation stage was selected for the experiments carried out, whereby all dissolved substances were collected in separator 1. The amount of substances depends on the selected extraction conditions (pressure and temperature), as well as on the solubility and the amount of substances contained in the raw material.

Process flow: The high-pressure laboratory system (HDL 4) was used for the experiments. This is a laboratory plant with one extractor and two separators. The plant is designed for an extraction pressure of up to 1000 bar and temperatures of up to 95°C. The extraction pressure is not higher than 1000 bar. Liquid CO₂ from the CO₂ tank is brought to extraction pressure by means of a pump and to extraction temperature by means of a heat exchanger. In the extractor, the now supercritical CO₂ flows through the raw material and is enriched with the soluble substances. After a pressure reduction, the mixture is separated into gaseous CO₂ and extract. The extract can be collected in the separator and removed from the plant. The now gaseous and uncharged CO₂ can then be liquefied again in the condenser and reused for the extraction cycle.

Performance: The raw material was presented to the extractor of the HDL 4 for extraction. Subsequently, an attempt was made to remove the valuable ingredients (such as terpenes and terpenoids) from the natural substance. Only one separation stage was used for the subsequent extract separation and the entire extract was collected in separator 1. Since the extract obtained is not flowable at -60 °C, conventional extract extraction via the outlet valve of the separator 1 is difficult to not possible. The extract had to be removed after each test run and additionally scraped off after opening the separator and partly solvents can be washed out.

### Example 2: Processing of olive tree material using the Venturi dryer

The original olive tree material is first subjected to a thermal-mechanical process in which the particle size of the raw material and its moisture content are specifically altered. The technical application result is a dehydrated, fine-grained, possibly also powdery product for direct further use in the process chain, possibly also for its intermediate storage.

### Example 3: Sterilisation process

Due to the natural contamination of the starting product with aerobic spore formers, various sterilization procedures were applied. Different sterilization temperatures were used to determine the optimal temperature for preserving the pharmaceutically active ingredients: 121°C and 134°C. The sterilization was carried out at different temperatures.

### Example 4: Comparison of active substances in cultivated and wild olive leaves, and the comminution method

The sampling of the wild and cultivated olive plant parts was carried out by hand in the region of Casa de Porros, Valdevaqueros, Tarifa, Spain. The plant parts were each processed as follows: A portion of fresh leaves, twigs, stem bark and root bark were dried in a Taprogge Venturi device, the other portion pulverized in a Thermomix. The sample material was stored in tightly closed containers protected from light at ambient temperature. For comparability, both samples were sieved to an identical particle size spectrum. Subsequently, Soxleth extraction, and chromatographic methods were performed to analyse the chemical composition of active agents both in cultivated and wild olive. A positive effect was found in Oxygen Radical Absorption Capacity (ORAC) testing; sampling of active substances after soxleth extraction revealed that higher yields in extraction were achieved for materials that had been comminuted in the Taprogge venturi device. Favorable influence on ORAC (Oxygen, Radical Absorption Capacity) was detected for materials comminuted by the Taprogge Venturi device, which, in turn, translates into positive conservation properties, and a particularly long shelf life. This was demonstrated by measuring the antioxidative activity of leaves and twigs processed by the Taprogge Venturi device method compared to the antioxidative activity of fresh samples over the course of 30 days while the value of the samples processed by the Taprogge Venturi device method remains constant. The results are shown in Table 2.

**Table 2: Comparison of the antioxidative activity of fresh samples and samples processed by the Taprogge Venturi device method over a 30-day period**

| | Fresh leaves and twigs (µmol Trolox equivalent/g of sample) | Leaves and twigs processed by VD (µmol Trolox equivalent/g of sample) |
|---|---|---|
| Day 1 | 521.98 | 502.14 (constant value) |
| Day 5 | 678.10 | |
| Day 10 | 678.77 | |
| Day 20 | 657.23 | |
| Day 30 | 618.49 | |

By comparing the active substances in the wild and cultivated olive, it can be seen that higher concentrations of active substances are present in the wild form of the olive tree. *Olea europaea* var. *europaea* and var. *sylvestris* differ in many aspects: The cultivated olive has larger fruits in a constant shape and is therefore more suitable for the production of olive oil. However, the tendency to produce larger fruits apparently means that the rest of the olive plant contains fewer active substances. The comparison of the two plants was made on the basis of already published data on the three most common species of cultivated olive in Andalusia: Arbequina, Hojiblanca and Picual. Fresh Acebuche olive leaves were examined for their profile of active substances (Table 3).

**Table 3: Concentrations of single substances in mg/g**

| **substances** | **var. *sylvestris* [mg/g]** | **var. *europaea*** | | |
|---|---|---|---|---|
| | | **Arbequina [mg/g]** | **Hojiblanca [mg/g]** | **Picual [mg/g]** |
| Oleuropein | 82.30 | 29.84 | 33.48 | 25.37 |
| Oleurosid | 42.86 | 13.34 | 8.11 | 8.10 |
| Ligstrosid | 12.55 | 3.85 | 3.48 | 3.25 |
| Verbacosid | 9.66 | 4.07 | 1.16 | 1.13 |
| Apigenin | 4.00 | 0.28 | 0.23 | 0.39 |
| Luteolin | 4.80 | 0.39 | 0.37 | 0.50 |
| Oleanolic acid | 8.32 | 4.72 | 2.57 | 3.29 |

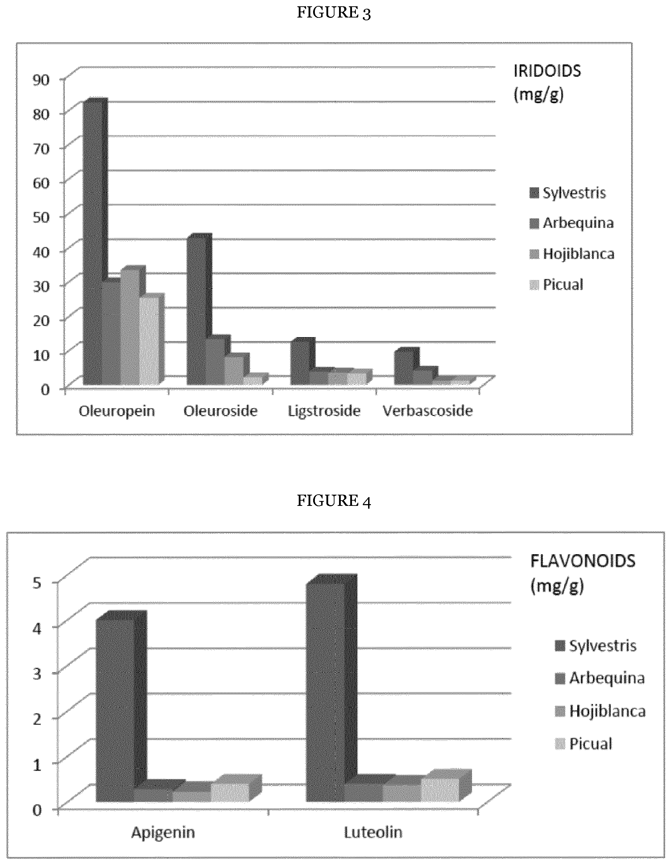

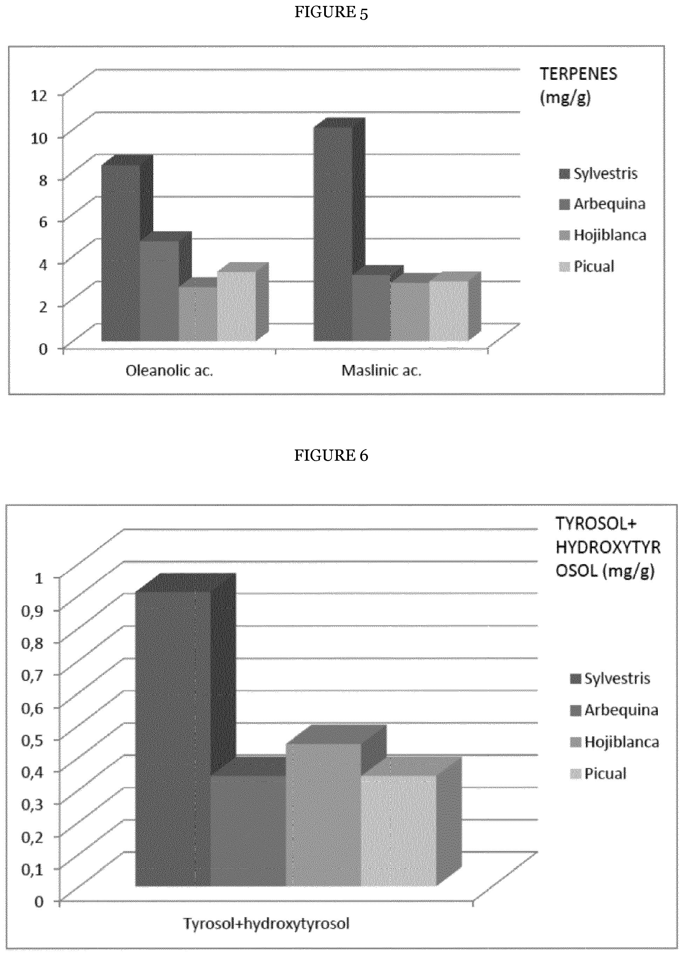

A comparison of active substances of different olive cultivars is shown in Figures 3 to 6, wherein Figure 3 shows the comparison of iridoids, Figure 4 shows the comparison flavinoids, Figure 5 shows the comparison of terpenes, and Figure 6 shows the comparison of tyrosol and hydroxytyrosol.

The following table 4 compares the percentage of active substances of acebuche vs. the cultivated olive.

**Table 4: Amounts of active substances in acebuche vs. cultivated olive as identified**

| **substances** | **var. *sylvestris*** | **var. *europaea*** | **% *sylvestris*/*europaea*** |
|---|---|---|---|
| Oleuropein | 82.30 | 29.53 | 278.7 % |
| Oleuroside | 42.86 | 9.85 | 435.1 % |
| Ligstroside | 12.55 | 3.53 | 355.5 % |
| Verbacoside | 9.66 | 2.12 | 455.7 % |
| Apigenine | 4.00 | 0.30 | 1333.3 % |
| Luteolin | 4.80 | 0.42 | 1142.8 % |
| Oleanolic acid | 8.32 | 3.53 | 235.7 % |
| Maslinic acid | 10.10 | 2.91 | 347.1 % |
| Tyrosol/Hydroxytyrosol | 0.91 | 0.37 | 246.0 % |

The comparison based on these nine substances shows that acebuche olive leaves have the highest concentrations of phenols and single phenols, especially flavinoids. It is apparent that from a pharmaceutical view wild olive (acebuche) and cultivated olive are absolutely distinct, and the wild olive is therefore the better raw material for a potentially therapeutic or cosmetic product.

### Example 5: Investigation of antimicrobial efficacy of olive or acebuche suspension

The aim of the investigations was to test the antimicrobial efficacy of olive or acebuche suspension after suspension in Kolliphor RH40 and water for injection (WFI) against the extended test germ spectrum (Table 5).

**Table 5: Test germ spectrum of the microbiological investigations of the present disclosure**

| Escherichia coli | | Staphylococcus aureus | | Pseudomonas aeruginosa | | Klebsiella pneumoniae | | Enterococcus | | Candida albicans | Acinetobacter baumannii | | Aspergillus brasiliensis |
|---|---|---|---|---|---|---|---|---|---|---|---|---|---|
| | ESBL | | MRSA | | ESBL | | subsp. pneumoniae | Hirae | Faecalis/VRE | | | | |
| ATCC 8739 | DSM 22312 | ATC 6538 | ATCC 29213 | ATCC 9027 | DSM 24600 | ATCC 10031 | CCUG 56233 | ATC C10541 | ATCCBAA 2317 | ATCC 10231 | ATCC 19606 | NCTC 13420 | ATCC 16404 |
| | | | | | | | KPC | | Vancomycin resistant | | | Carbapenem resistant | |

Performance: Preparation of the test germ suspension: The test bacteria were cultivated with a maximum passage number of 5. The test bacteria suspensions were adjusted to approximately 1000 CFU/0.1 ml in sterile 0.9% NaCl solution.

Sample preparation: 3.00 g olive extract in 1.01 g Kolliphor RH40 were heated for approximately 5 min. at 45°C in a water bath, then for approximately 15 min. at 80°C in a water bath and shaken by hand. The olive extract and Kolliphor RH40 were mixed together with 15 ml WFI, resulting in a very viscous, dark green to dark brown suspension that could not be pipetted. The next day the stock solution was heated again at 80°C in a water bath and another 5 ml WFI heated to 80°C was added. The stock solution was again heated to 80°C for approximately 5 minutes. Water bath warmed up and shaken approx. 1 min. by hand. The result was a stock solution containing 125 mg olive extract per ml and 4.2% Kolliphor RH40. After this processing, the olive extract was available as a homogeneous and pipettable suspension with a dark brown to dark green color.

Evaluation of the antimicrobial efficacy of olive extract after preparation of stock solutions: For each test germ 1 ml of the stock solution was converted to 1 ml of double concentrated CaSo bouillon and Sab bouillon (*C*. *albicans*). The preparations were then inoculated with 0.1 ml each of the test germ suspensions adjusted to approx. 1000 CFU/0.1 ml. The bacterial count in the test germ suspensions was checked for blood agar and sab-agar by means of the spatula method. All culture media preparations were incubated for a maximum of 72 hours at (30-35°C). After 24h, 48h, and 72 hours incubation subcultures of all 0.1 ml batches were performed by streaking on blood agar plates and Sab-agar respectively. The agar plates were tested at 30-35°C for 24 h - 48 h (yeast) aerobically incubated. Positive controls: 1 ml WFI were added to 1 ml double concentrated CaSo bouillon or Sab bouillon (*C*. *albicans*) and then inoculated with 0.1 ml each of the test germ suspensions adjusted to approx. 1000 KBE/0.1 ml in single determination. The positive controls were used as a reference for the growth of test bacteria in the double concentrated culture medium after 1:2 dilutions. The results are presented in the following tables.

**Table 6: Results of the antimicrobial efficacy of olive suspensions with the test bacterium C. albicans ATCC10231; Inoculum: effectively inoculated bacterial count per test batch: 1160 CFU = 580 CFU/ml culture medium batch**

| **sample ID** | **Test concentration in the nutrient medium** | **Results** | | |
|---|---|---|---|---|
| | | **Growth after 24 h** | **Growth after 48 h** | **Growth after 72 h** |
| positive control | ./. | + | + | + |
| | | After subculture growth (layer) | After subculture growth (layer) | After subculture growth (layer) |
| stock solution (125 mg/ml product) | stock solution (62.5 mg/ml product) | P | P | P |
| | | not visually recognizable/**No growth after subculture** | not visually recognizable/**No growth after subculture** | not visually recognizable/**No growth after subculture** |

| | | | | |
|---|---|---|---|---|
| + = Growth of the test germ in the form of visually visible turbidity in the nutrient media preparations P = Product turbidity, no visual assessment of the growth of the test germ possible Subcultures (SC) by streaking of 0.1 ml each of the nutrient media preparation | | | | |

**Table 7: Results of the antimicrobial efficacy of olive suspension with the test bacterium Ent. faecium ATCC BAA2317; Inoculum: effectively inoculated bacterial count per test batch: 1810 CFU = 905 CFU / ml culture medium batch**

| **sample ID** | **Test concentration in the nutrient medium** | **Results** | | |
|---|---|---|---|---|
| | | **Growth after 24 h** | **Growth after 48 h** | **Growth after 72 h** |
| positive control | ./. | + | + | + |
| | | After subculture growth (layer) | After subculture growth (layer) | After subculture growth (layer) |
| stock solution (125 mg/ml product) | stock solution (62,5 mg/ml product) | P | P | P |
| | | not visually recognizable/**No growth after subculture** | not visually recognizable/**No growth after subculture** | not visually recognizable/**No growth after subculture** |

| | | | | |
|---|---|---|---|---|
| + = Growth of the test germ in the form of visually visible turbidity in the nutrient media preparations P = Product turbidity, no visual assessment of the growth of the test germ possible Subcultures (SC) by streaking of 0.1 ml each of the nutrient media preparation | | | | |

**Table 8: Results of the antimicrobial efficacy of olive suspension with the test bacterium Ent. hirae ATCC 10541; Inoculum: effectively inoculated bacterial count per test batch: 760 CFU = 380 CFU / ml culture medium batch**

| **sample ID** | **Test concentration in the nutrient medium** | **Results** | | |
|---|---|---|---|---|
| | | **Growth after 24 h** | **Growth after 48 h** | **Growth after 72 h** |
| positive control | ./. | + | + | + |
| | | After subculture growth (layer) | After subculture growth (layer) | After subculture growth (layer) |
| stock solution (125 mg/ml product) | stock solution (62.5 mg/ml product) | P | P | P |
| | | not visually recognizable/**No growth after subculture** | not visually recognizable/**No growth after subculture** | not visually recognizable/**No growth after subculture** |

| | | | | |
|---|---|---|---|---|
| + = Growth of the test germ in the form of visually visible turbidity in the nutrient media preparations P = Product turbidity, no visual assessment of the growth of the test germ possible Subcultures (SC) by streaking of 0.1 ml each of the nutrient media preparation | | | | |

**Table 9: Results of the antimicrobial efficacy of olive suspension with the test bacterium Kleb. pneu. subsp. Pneumoniae CCUG 56233; Inoculum: effectively inoculated bacterial count per test batch: 1200 CFU = 600 CFU / ml culture medium batch**

| **sample ID** | **Test concentration in the nutrient medium** | **Results** | | |
|---|---|---|---|---|
| | | **Growth after 24 h** | **Growth after 48 h** | **Growth after 72 h** |
| positive control | ./. | + | + | + |
| | | After subculture growth (layer) | After subculture growth (layer) | After subculture growth (layer) |
| stock solution (125 mg/ml product) | stock solution (62.5 mg/ml product) | P | P | P |
| | | not visually recognizable/**No growth after subculture** | not visually recognizable/**No growth after subculture** | not visually recognizable/**No growth after subculture** |

| | | | | |
|---|---|---|---|---|
| + = Growth of the test germ in the form of visually visible turbidity in the nutrient media preparations P = Product turbidity, no visual assessment of the growth of the test germ possible Subcultures (SC) by streaking of 0.1 ml each of the nutrient media preparation | | | | |

**Table 10: Results of the antimicrobial efficacy of olive suspension with the test bacterium Kleb. pneu. subsp. pneumoniae ATCC 10031; Inoculum: effectively inoculated bacterial count per test batch: 740 CFU = 370 CFU / ml culture medium batch**

| **sample ID** | **Test concentration in the nutrient medium** | **Results** | | |
|---|---|---|---|---|
| | | **Growth after 24 h** | **Growth after 48 h** | **Growth after 72 h** |
| positive control | ./. | + | + | + |
| | | After subculture growth (layer) | After subculture growth (layer) | After subculture growth (layer) |
| stock solution (125 mg/ml product) | stock solution (62.5 mg/ml product) | P | P | P |
| | | not visually recognizable/**No growth after subculture** | not visually recognizable/**No growth after subculture** | not visually recognizable/**No growth after subculture** |

| | | | | |
|---|---|---|---|---|
| + = Growth of the test germ in the form of visually visible turbidity in the nutrient media preparations P = Product turbidity, no visual assessment of the growth of the test germ possible Subcultures (SC) by streaking of 0.1 ml each of the nutrient media preparation | | | | |

**Table 11: Results of the antimicrobial efficacy of olive suspension with the test bacterium P. aeruginosa ATCC 9027; Inoculum: effectively inoculated bacterial count per test batch: 640 CFU = 320 CFU / ml culture medium batch**

| **sample ID** | **Test concentration in the nutrient medium** | **Results** | | |
|---|---|---|---|---|
| | | **Growth after 24 h** | **Growth after 48 h** | **Growth after 72 h** |
| positive control | ./. | + | + | + |
| | | After subculture growth (layer) | After subculture growth (layer) | After subculture growth (layer) |
| stock solution (125 mg/ml product) | stock solution (62.5 mg/ml product) | P | P | P |
| | | not visually recognizable/**No growth after subculture** | not visually recognizable/**No growth after subculture** | not visually recognizable/**No growth after subculture** |

| | | | | |
|---|---|---|---|---|
| + = Growth of the test germ in the form of visually visible turbidity in the nutrient media preparations P = Product turbidity, no visual assessment of the growth of the test germ possible Subcultures (SC) by streaking of 0.1 ml each of the nutrient media preparation | | | | |

**Table 12: Results of the antimicrobial efficacy of olive suspension with the test bacterium P. aeruginosa ESBL DSM 24600; Inoculum: effectively inoculated bacterial count per test batch: 680 CFU = 340 CFU / ml culture medium batch**

| **sample ID** | **Test concentration in the nutrient medium** | **Results** | | |
|---|---|---|---|---|
| | | **Growth after 24 h** | **Growth after 48 h** | **Growth after 72 h** |
| positive control | ./. | + | + | + |
| | | After subculture growth (layer) | After subculture growth (layer) | After subculture growth (layer) |
| stock solution (125 mg/ml product) | stock solution (62.5 mg/ml product) | P | P | P |
| | | not visually recognizable/**No growth after subculture** | not visually recognizable/**No growth after subculture** | not visually recognizable/**No growth after subculture** |

| | | | | |
|---|---|---|---|---|
| + = Growth of the test germ in the form of visually visible turbidity in the nutrient media preparations P = Product turbidity, no visual assessment of the growth of the test germ possible Subcultures (SC) by streaking of 0.1 ml each of the nutrient media preparation | | | | |

**Table 13: Results of the antimicrobial efficacy of olive suspension with the test bacterium E. coli ATCC 8739; Inoculum: effectively inoculated bacterial count per test batch: 860 CFU = 430 CFU / ml culture medium batch**

| **sample ID** | **Test concentration in the nutrient medium** | **Results** | | |
|---|---|---|---|---|
| | | **Growth after 24 h** | **Growth after 48 h** | **Growth after 72 h** |
| positive control | ./. | + | + | + |
| | | After subculture growth (layer) | After subculture growth (layer) | After subculture growth (layer) |
| stock solution (125 mg/ml product) | stock solution (62.5 mg/ml product) | P | P | P |
| | | not visually recognizable/**No growth after subculture** | not visually recognizable/**No growth after subculture** | not visually recognizable/**No growth after subculture** |

| | | | | |
|---|---|---|---|---|
| + = Growth of the test germ in the form of visually visible turbidity in the nutrient media preparations P = Product turbidity, no visual assessment of the growth of the test germ possible Subcultures (SC) by streaking of 0.1 ml each of the nutrient media preparation | | | | |

**Table 14: Results of the antimicrobial efficacy of olive suspension with the test bacterium E. coli DSM 22312; Inoculum: effectively inoculated bacterial count per test batch: 670 CFU = 435 CFU / ml culture medium batch**

| **sample ID** | **Test concentration in the nutrient medium** | **Results** | | |
|---|---|---|---|---|
| | | **Growth after 24 h** | **Growth after 48 h** | **Growth after 72 h** |
| positive control | ./. | + | + | + |
| | | After subculture growth (layer) | After subculture growth (layer) | After subculture growth (layer) |
| stock solution (125 mg/ml product) | stock solution (62.5 mg/ml product) | P | P | P |
| | | not visually recognizable/**No growth after subculture** | not visually recognizable/**No growth after subculture** | not visually recognizable/**No growth after subculture** |

| | | | | |
|---|---|---|---|---|
| + = Growth of the test germ in the form of visually visible turbidity in the nutrient media preparations P = Product turbidity, no visual assessment of the growth of the test germ possible Subcultures (SC) by streaking of 0.1 ml each of the nutrient media preparation | | | | |

**Table 15: Results of the antimicrobial efficacy of olive suspension with the test bacterium S. aureus ATCC 6538; Inoculum: effectively inoculated bacterial count per test batch: 680 CFU = 340 CFU / ml culture medium batch**

| **sample ID** | **Test concentration in the nutrient medium** | **Results** | | |
|---|---|---|---|---|
| | | **Growth after 24 h** | **Growth after 48 h** | **Growth after 72 h** |
| positive control | ./. | + | + | + |
| | | After subculture growth (layer) | After subculture growth (layer) | After subculture growth (layer) |
| stock solution (125 mg/ml product) | stock solution (62.5 mg/ml product) | P | P | P |
| | | not visually recognizable/**No growth after subculture** | not visually recognizable/**No growth after subculture** | not visually recognizable/**No growth after subculture** |

| | | | | |
|---|---|---|---|---|
| + = Growth of the test germ in the form of visually visible turbidity in the nutrient media preparations P = Product turbidity, no visual assessment of the growth of the test germ possible Subcultures (SC) by streaking of 0.1 ml each of the nutrient media preparation | | | | |

**Table 16: Results of the antimicrobial efficacy of olive suspension with the test bacterium S. aureus supsp. aureus ATCC 29213; Inoculum: effectively inoculated bacterial count per test batch: 2020 CFU = 1010 CFU / ml culture medium batch**

| **sample ID** | **Test concentration in the nutrient medium** | **Results** | | |
|---|---|---|---|---|
| | | **Growth after 24 h** | **Growth after 48 h** | **Growth after 72 h** |
| positive control | ./. | + | + | + |
| | | After subculture growth (layer) | After subculture growth (layer) | After subculture growth (layer) |
| stock solution (125 mg/ml product) | stock solution (62.5 mg/ml product) | P | P | P |
| | | not visually recognizable/**No growth after subculture** | not visually recognizable/**No growth after subculture** | not visually recognizable/**No growth after subculture** |

| | | | | |
|---|---|---|---|---|
| + = Growth of the test germ in the form of visually visible turbidity in the nutrient media preparations P = Product turbidity, no visual assessment of the growth of the test germ possible Subcultures (SC) by streaking of 0.1 ml each of the nutrient media preparation | | | | |

For investigation of two further test germs, the bacteria *Acinetobacter baumannii* and *Aspergillus brasiliensis,* the Kolliphor RH40 content of the stock solution was reduced by 50% from 4.2% to 2.1%. In addition to this origin stock solution A1 with the olive extract in a concentration of 127.5 mg/ml a 1:2 dilution with water for injection was prepared so that the olive extract had a concentration of 63.8 mg/ml (stock solution A2).

**Table 17: Results of the antimicrobial efficacy of olive suspension with the test bacterium A. baumannii ATCC 19606; Inoculum: effectively inoculated bacterial count per test batch: 4000 CFU = 2000 CFU / ml culture medium batch**

| **sample ID** | **Test concentration in the nutrient medium** | **Results** | | |
|---|---|---|---|---|
| | | **Growth after 24 h** | **Growth after 48 h** | **Growth after 72 h** |
| positive control | ./. | + | + | + |
| | | After subculture growth (layer) | After subculture growth (layer) | After subculture growth (layer) |
| stock solution A1 (127.5 mg/ml product) | stock solution A1 (63.8 mg/ml product) | P | P | P |
| | | not visually recognizable/**No growth after subculture** | not visually recognizable/**No growth after subculture** | not visually recognizable/**No growth after subculture** |
| stock solution A2 (63.8 mg/ml product) | stock solution A2 (31.9 mg/ml product) | P | P | P |
| | | not visually recognizable/**No growth after subculture** | not visually recognizable/**growth after subculture, comparable with control** | not visually recognizable/**growth after subculture, comparable with control** |

| | | | | |
|---|---|---|---|---|
| = Growth of the test germ in the form of visually visible turbidity in the nutrient media preparations P = Product turbidity, no visual assessment of the growth of the test germ possible Subcultures (SC) by streaking of 0.1 ml each of the nutrient media preparation | | | | |

**Table 18: Results of the antimicrobial efficacy of olive suspension with the test bacterium A. baumannii NCTC 13420; Inoculum: effectively inoculated bacterial count per test batch: 890 CFU = 445 CFU / ml culture medium batch**

| **sample ID** | **Test concentration in the nutrient medium** | **Results** | | |
|---|---|---|---|---|
| | | **Growth after 24 h** | **Growth after 48 h** | **Growth after 72 h** |
| positive control | ./. | + | + | + |
| | | After subculture growth (layer) | After subculture growth (layer) | After subculture growth (layer) |
| stock solution A1 (127.5 mg/ml product) | stock solution A1 (63.8 mg/ml product) | P | P | P |
| | | not visually recognizable/**No growth after subculture** | not visually recognizable/**No growth after subculture** | not visually recognizable/**No growth after subculture** |
| stock solution A2 (63.8 mg/ml product) | stock solution A2 (31.9 mg/ml product) | P | P | P |
| | | not visually recognizable/**No growth after subculture** | not visually recognizable/**No growth after subculture** | not visually recognizable/**No growth after subculture** |

| | | | | |
|---|---|---|---|---|
| + = Growth of the test germ in the form of visually visible turbidity in the nutrient media preparations P = Product turbidity, no visual assessment of the growth of the test germ possible Subcultures (SC) by streaking of 0.1 ml each of the nutrient media preparation | | | | |

**Table 19: Results of the antimicrobial efficacy of olive suspension with the test bacterium A. brasiliensis ATCC 16404; Inoculum: effectively inoculated bacterial count per test batch: 620 CFU = 310 CFU / ml culture medium batch**

| **sample ID** | **Test concentration in the nutrient medium** | **Results** | | |
|---|---|---|---|---|
| | | **Growth after 24 h** | **Growth after 48 h** | **Growth after 72 h** |
| positive control | ./. | + | + | + |
| | | After subculture growth (4 CFU) | After subculture growth (layer) | After subculture growth (layer) |
| stock solution A1 (127.5 mg/ml product) | stock solution A1 (63.8 mg/ml product) | P | P | P |
| | | not visually recognizable/**After subculture growth of approx.28 CFU/0.1 ml, comparable with control** | not visually recognizable/**After subculture growth of 6 CFU/0.1 ml, not comparable with control** | not visually recognizable/**After subculture growth of 2 CFU/0.1 ml, not comparable with control** |
| stock solution A2 (63.8 mg/ml product) | stock solution A2 (31.9 mg/ml product) | P | P | P |
| | | not visually recognizable/**After subculture growth of 9 CFU/0.1 ml, comparable with control** | not visually recognizable/**After subculture growth of 2 CFU/0.1 ml, comparable with control** | not visually recognizable/**After subculture growth of 1 CFU/0.1 ml, comparable with control** |

| | | | | |
|---|---|---|---|---|
| + = Growth of the test germ in the form of visually visible turbidity in the nutrient media preparations P = Product turbidity, no visual assessment of the growth of the test germ possible Subcultures (SC) by streaking of 0.1 ml each of the nutrient media preparation | | | | |

As the tests show, no growth of test bacteria, with the exception of the test bacterium *A*. *brasiliensis* ATCC 16404, can be detected after 72 hours of incubation of the nutrient media preparations after carrying out the corresponding subcultures. Thus, a good antimicrobial efficacy of the olive extract in a concentration of 62.5 mg/ml or 63.8 mg/ml respectively can be demonstrated under the test conditions. For the test germ *A. brasiliensis* in the presence of the olive suspension from stock solution A1 and stock solution A2, a few isolated surviving live spores are still detectable throughout the test, but the reduction of CFU is also significant here.

### Example 6: Investigation of antimicrobial efficacy of olive or acebuche gel

Performance: A colloidal gel with Kelcogel CG-HA was produced from olive leaf powder. For this purpose, 0.1 g Kelcogel CG-HA was dissolved in 100 ml WFI by heating to 85-90°C and then 15 g olive leaf powder was added while stirring until a homogeneous suspension was visually formed. After cooling in the ice bath, a colloidal olive leaf powder gel was obtained. Aliquots of 10 g gel were inoculated with the adjusted test germ suspension (10⁵ - 10⁶ CFU/g) and tested for germ count according to the test times listed in the table 15.

**Table 20: Number of CFU according to the test time.**

| **Test germs** | **Initial load CFU/g** | **t₀ CFU/g** | **t=6 min CFU/g** | **t=12 min CFU/g** | **t=30 min CFU/g** | **t=60 min CFU/g** |
|---|---|---|---|---|---|---|
| ***Staphylococcus aureus* ATCC 6538** | 4.0 x 10⁵ | 2.7 x 10³ | 1.0 x 10² | <100 | <100 | <100 |
| ***Pseudomonas aeruginosa* ATCC 15442** | 7.5 x 10⁵ | 5.6 x 10³ | 4.0 x 10² | <100 | <100 | <100 |
| ***Escherichia coli* ATCC 8739** | 6.3 x 10⁵ | <100 | <100 | <100 | <100 | <100 |

After 12 minutes, E. coli already showed a very good efficacy at the t₀ h-value and *S. aureus* and *P. aeruginosa* below the detection limit of 100 CFU per gram, and proves the overall very good antimicrobial efficacy within a short exposure period.

### Example 7: Investigation of antimicrobial efficacy of olive or acebuche gel

Performance: A colloidal gel with Kelcogel CG-HA was produced from olive leaf powder. For this purpose, 0.1 g Kelcogel CG-HA was dissolved in 100 ml WFI by heating to 85-90°C and then 15 g olive leaf powder was added while stirring until a homogeneous suspension was visually formed. After cooling in the ice bath, a colloidal olive leaf powder gel was obtained. Aliquots of 10 g gel were inoculated with the adjusted test germ suspension (105 - 10⁶ CFU/g) and tested for germ count according to the test times listed in the table 21.

**Table 21: Number of CFU according to the test time.**

| **Test germs** | **Initial load CFU/g** | **t₀ CFU/g** | **t=6 min CFU/g** | **t=12 min CFU/g** | **t=30 min CFU/g** | **t=60 min CFU/g** |
|---|---|---|---|---|---|---|
| ***Staphylococcus aureus* ATCC 6538** | 4.0 x 10⁵ | 6.1 x 10³ | 6.1 x 10² | <100 | <100 | <100 |
| ***Pseudomonas aeruginosa* ATCC 15442** | 7.5 x 10⁵ | 5.9 x 10³ | 6.1 x 10² | 1.0 x 10² | <100 | <100 |
| ***Escherichia coli* ATCC 8739** | 6.3 x 10⁵ | 3.0 x 10² | <100 | <100 | <100 | <100 |

Accordingly, *E. coli* shows very good efficacy after 6 minutes and *S. aureus* after 12 minutes up to below the detection limit of 100 CFU per gram. For *P. aeruginosa* the bacterial count is reduced to below the detection limit after 30 minutes and proves the overall very good antimicrobial efficacy within a short exposure period.

### Example 8: Investigation of antimicrobial efficacy of olive or acebuche composition with dough pieces

Performance: Dough pieces were prepared per test time by mixing 500 mg sample with 1 ml WFI. After homogenization, 0.5 ml of adjusted germ suspension was added and homogeneously mixed into the dough. Afterwards both test germs showed a very good efficacy already after 6 hours reaction time at room temperature with the untreated as well as with the 15 minutes at 121°C and 30 minutes at 130°C steam-sterilized product olive leaf in powder form. (Table 22)

**Table 22: Number of CFU according to the test time.**

| **Test germ** | **Initial load CFU/dough pieces** | **t₀ CFU/dough pieces** | **6 h CFU/dough pieces** | **24 h CFU/dough pieces** |
|---|---|---|---|---|
| ***Staphylococcus aureus*** MRSA-strain ATCC 29213 Untreated olive leaf powder | 2.0 x 10⁵ | 1.8 x 10⁵ | <100 | <100 |
| ***Staphylococcus aureus*** MRSA-strain ATCC 29213 olive leaf powder sterilized at 121°C for 15 min. | 2.0 x 10⁵ | 2.4 x 10⁵ | <100 | <100 |
| ***Staphylococcus aureus*** MRSA-strain ATCC 29213 olive leaf powder sterilized at 130°C for 30 min. | 2.0 x 10⁵ | 3.4 x 10⁵ | <100 | <100 |

The untreated olive leaf dough pieces show a natural contamination of the order of 1.4 x 10³ CFU - 2.1 x 10³ CFU per 500 mg dough piece with various aerobic spore formers, which was not killed within the test period after 6 hours and 24 hours respectively. In the sterilized test samples at 121°C for 15 minutes, the dough pieces showed a natural contamination in the range of 1.1 x 103 CFU - 2.2 x 102 CFU per 500 mg. Both sterilized test specimens were sterilized for 30 minutes at 130°C and no contamination germs were detected.

### Example 9: Investigation of antimicrobial efficacy of olive or acebuche composition with dough pieces

Performance: Dough pieces were prepared by mixing 500 mg olive substrate with 1 ml WFI per test germ and test time. After homogenization, 0.5 ml of adjusted germ suspension was added and homogeneously mixed into the dough. Afterwards both test germs showed a very good efficacy already after 24 hours reaction time at room temperature both at untreated and at 15 minutes at 121°C and 30 minutes at 130°C steam sterilized olive leaf. (Table 23)

**Table 23: Number of CFU according to the test time.**

| **Test germ** | **Initial load CFU/dough pieces** | **t₀ CFU/dough pieces** | **24 h CFU/dough pieces** |
|---|---|---|---|
| ***Escherichia coli* ATCC** 8739 Untreated olive leaf powder | 1.3 x 10⁵ | 1.3 x 10⁵ | <100 |
| ***Pseudomonas aeruginosa*** ATCC 9027 Untreated olive leaf powder | 2.2 x 10⁵ | 2.1 x 10⁵ | <100 |
| ***Staphylococcus aureus*** ATCC 6538 Untreated olive leaf powder | 1.4 x 10⁵ | 1.8 x 10⁵ | <100 |
| ***Escherichia coli*** ATCC 8739 olive leaf powder sterilized at 121°C for 15 min. | 1.3 x 10⁵ | 1.3 x 10⁵ | <100 |
| ***Pseudomonas aeruginosa*** ATCC 9027 olive leaf powder sterilized at 121°C for 15 min. | 2.2 x 10⁵ | 2.2 x 10⁵ | <100 |
| ***Staphylococcus aureus*** ATCC 6538 olive leaf powder sterilized at 121°C for 15 min. | 1.4 x 10⁵ | 2.4 x 10⁵ | <100 |
| ***Escherichia coli*** ATCC 8739 olive leaf powder sterilized at 130°C for 30 min. | 1.3 x 10⁵ | 2.3 x 10⁵ | <100 |
| ***Pseudomonas aeruginosa*** ATCC 9027 olive leaf powder sterilized at 130°C for 30 min. | 2.2 x 10⁵ | 1.6 x 10⁵ | <100 |
| ***Staphylococcus aureus*** ATCC 6538 olive leaf powder sterilized at 130°C for 30 min. | 1.4 x 10⁵ | 1.9 x 10⁵ | <100 |

The untreated olive leaf dough pieces show a natural contamination of the order of 2.9 x 10⁴ CFU per 500 mg dough piece with various aerobic spore formers, which were not killed within the test period after 24 hours. No contamination germs are detectable in the sterilized test samples.

### Example 10: Investigation of antimicrobial efficacy of olive or acebuche composition with dough pieces

Performance: Dough pieces were prepared by mixing 500 mg olive substrate with 1 ml WFI per test germ and test time. After homogenization, 0.5 ml of adjusted germ suspension was added and homogeneously mixed into the dough. Afterwards, both test germs showed a very good efficacy after 24 hours reaction time at room temperature both at untreated and at 15 minutes at 121°C and 30 minutes at 130°C steam-sterilized product bark/strain in powder form. (Table 24)

**Table 24: Number of CFU according to the test time.**

| **Test germ** | **Initial load CFU/dough pieces** | **t₀ CFU/dough pieces** | **24 h CFU/dough pieces** |
|---|---|---|---|
| ***Escherichia coli*** ATCC 8739 Untreated bark/strain powder | 1.4 x 10⁵ | 1.2 x 10⁵ | <100 |
| ***Pseudomonas aeruginosa*** ATCC 9027 Untreated bark/strain powder | 1.3 x 10⁵ | 2.7 X 10⁵ | <100 |
| ***Staphylococcus aureus*** ATCC 6538 Untreated bark/strain powder | 2.1 x 10⁵ | 5.3 x 10⁵ | <100 |
| ***Escherichia coli*** ATCC 8739 bark/strain powder sterilized at 121°C for 15 min. | 1.4 x 10⁵ | 1.3 x 10⁵ | <100 |
| ***Pseudomonas aeruginosa*** ATCC 9027 bark/strain powder sterilized at 121°C for 15 min. | 1.3 x 10⁵ | 2.0 x 10⁵ | <100 |
| ***Staphylococcus aureus*** ATCC 6538 bark/strain powder sterilized at 121°C for 15 min. | 2.1 x 10⁵ | 1.8 x 10⁵ | <100 |
| ***Escherichia coli*** ATCC 8739 bark/strain powder sterilized at 130°C for 30 min. | 1.4 x 10⁵ | 1.6 x 10⁵ | <100 |
| ***Pseudomonas aeruginosa*** ATCC 9027 bark/strain powder sterilized at 130°C for 30 min. | 1.3 x 10⁵ | 1.8 x 10⁵ | <100 |
| ***Staphylococcus aureus*** ATCC 6538 bark/strain powder sterilized at 130°C for 30 min. | 2.1 x 10⁵ | 1.2 x 10⁵ | <100 |

The untreated bark/strain in powder form showed a natural contamination of the order of 4.4 x 10² CFU per 500 mg dough with various aerobic spore formers, which was not killed within the test period after 24 hours. Contamination germs of 1.1 x 10² CFU/dough piece are still detectable in the test specimen sterilized at 121°C. The test specimen is also sterilized at 121°C. In the test specimen sterilized at 130°C no contamination germs are detectable.

### Example 11: Investigation of antimicrobial efficacy of olive or acebuche composition with dough pieces

Performance: Dough pieces were prepared by mixing 500 mg olive substrate with 1 ml WFI per test germ and test time. After homogenization, 0.5 ml of adjusted germ suspension was added and homogeneously mixed into the dough. Afterwards both test germs showed a very good efficacy already after 24 hours reaction time at room temperature both at untreated and at 15 minutes at 121°C and 30 minutes at 130°C steam-sterilized product bark/root in powder form. (Table 25)

**Table 25: Number of CFU according to the test time**

| **Test germ** | **Initial load CFU/dough pieces** | **t₀ CFU/dough pieces** | **24 h CFU/dough pieces** |
|---|---|---|---|
| ***Escherichia coli*** ATCC 8739 Untreated bark/root powder | 1.4 x 10⁵ | 1.8 x 10⁵ | <100 |
| ***Pseudomonas aeruginosa*** ATCC 9027 Untreated bark/root powder | 1.3 x 10⁵ | 3.3 x 10⁵ | <100 |
| ***Staphylococcus aureus*** ATCC 6538 Untreated bark/root powder | 2.1 x 10⁵ | 1.1 x 10⁵ | <100 |
| ***Escherichia coli*** ATCC 8739 Bark/root powder sterilized at 121°C for 15 min. | 1.4 x 10⁵ | 1.4 x 10⁵ | <100 |
| ***Pseudomonas aeruginosa*** ATCC 9027 bark/root powder sterilized at 121°C for 15 min. | 1.3 x 10⁵ | 2.4 x 10⁵ | <100 |
| ***Staphylococcus aureus*** ATCC 6538 bark/root powder sterilized at 121°C for 15 min. | 2.1 x 10⁵ | 3.1 x 10⁵ | <100 |
| ***Escherichia coli*** ATCC 8739 bark/root powder sterilized at 130°C for 30 min. | 1.4 x 10⁵ | 2.3 x 10⁵ | <100 |
| ***Pseudomonas aeruginosa*** ATCC 9027 bark/root powder sterilized at 130°C for 30 min. | 1.3 x 10⁵ | 2.3 x 10⁵ | <100 |
| ***Staphylococcus aureus*** ATCC 6538 bark/root powder sterilized at 130°C for 30 min. | 2.1 x 10⁵ | 1.3 x 10⁵ | <100 |

The untreated bark/root in powder form showed a natural contamination in the range of 1.1 x 10⁶ CFU per 500 mg dough with various aerobic spore formers, which was not killed within the test period after 24 hours. In the test samples sterilized at 121°C and 130°C, contamination germs of 1.8 x 10³ CFU/dough piece and 8.8 x 10² CFU/ dough piece are still detectable.

### REFERENCES AS CITED

**1.** AG, B.M. (2018)."Multiresistente Erreger." 2018, from https://www.bbraun.de/de/produkte-undtherapien/hyiene/multiresistente-erreger.html
**2.** Centers for Disease, Control and Prevention (2011). Surveillance for Foodborne Disease Outbreaks - United States, 2008, MMWR, Morbidity and Mortality Weekly Report. 60: 1197-1202.
**3.** Effect, B. N. (2017). "Olivenblattextrakt Forschung." Retrieved 10.10.2017, from http://www.best-natural-effect.com/info/5/olivenblattextrakt-forschung.html.
**4.** Fleming, H. P., et al. (1973). "Antimicrobial properties of oleuropein and products of its hydrolysis from green olives." Appl Microbiol 26(5): 777-782.
**5.** Guinda, A., et al. (2010). "Pentacyclic triterpenoids from olive fruit and leaf." J Agric Food Chem 58(17): 9685-9691.
**6.** Iming, S. (2005). Olivenblattextrakte - Altbewährte Heilmittel in der Praxis. Wien, Double-U GmbH.
**7.** Khan, M. Y., et al. (2007). "Olea europaea: A phyto-pharmacological review." Pharmacognosy Reviews 1(1): 114-118.
**8.** Krämer, J. (2007). Lebensmittel-Mikrobiologie, Ulmer.
**9.** Lieberei, R. R., C. (2007). Nutzpflanzenkunde. Stuttgart, Thieme Verlag.
**10.** Liu, Y., et al. (2017). "Assessment of the Antimicrobial Activity of Olive Leaf Extract Against Foodborne Bacterial Pathogens." Front Microbiol 8: 113.
**11.** Stiti, N., et al. (2007). "Formation of triterpenoids throughout Olea europaea fruit ontogeny." Lipids 42(1): 55-67.
**12.** WHO, W.H.O. (2018). "Antibiotikaresistenz." from http://www.euro.who.int/de/health-topics/disease-prevention/antimicrobial-resistance/antibiotic-resistance.
**13.** Ahmed et al. (2014); "Antibacterial effect of olive (Olea europaea L.) leaves extract in raw peeled undeveined shrimp (Penaeus semisulcatus)
**14.** US 7,429,008 B2; SYSTEM AND METHOD FOR PULVERIZING AND EXTRACTING MOISTURE; Graham et al.; 2008
**15**. US 7,500,830 B2 SYSTEM AND METHOD FOR PULVERIZING AND EXTRACTING MOISTURE; Graham et al.; 2009
**16.** US 7,909,577 B2 SYSTEM AND METHOD FOR PULVERIZING AND EXTRACTING MOISTURE; Graham et al.; 2011
**17.** WO 2013/052583 A2 SYSTEMS AND METHODS FOR CONVERTWG SEWAGE SLUDGE WTO A COMBUSTIBLE FUEL; New et al.; 2013
**18.** WO 2013/075003 A1; EGGSHELL POWDER COMPOSITIONS AND METHODS OF PRODUCING EGGSHELL POWDER COMPOSITIONS; Liu et al., 2013

## Claims

1. A method for preparing an olive (*Olea europaea subsp. europaea*), preferably acebuche (Olea europaea subsp. europaea var. sylvestris) composition, comprising the steps of:
a) providing parts of an olive or acebuche plant; and
b) removing moisture from said olive or acebuche parts by dewatering or drying of said olive or acebuche parts; and
c) comminution of said olive or acebuche parts of step b) by chopping, grinding, milling, pulverizing or other methods of comminution of said olive or acebuche parts to obtain a pulverized olive or acebuche;
d) suitably sterilizing said pulverized olive or acebuche, preferably at a temperature of 121°C or 134°C to obtain a sterilized pulverized olive or acebuche; and, as may be desired,
e) blending of said sterilized pulverized olive or acebuche with water and a thickening agent to obtain an antibiotically or antifungally active olive or acebuche gel; or, as may be desired; or
f) extracting an antibiotically or antifungally active agent from the pulverized olive or acebuche from step c) by extraction, preferably CO₂ extraction; suspending of said active agent in a non-toxic solubilizer and emulsifying agent to obtain an antibiotically or antifungally active olive or acebuche suspension.

2. The method according to claim 1, wherein steps b) and c) are performed simultaneously by subjecting said olive or acebuche to forces generated by dynamic airflow, preferably by passing said olive or acebuche together with air, preferably pre-heated air, through a Venturi nozzle.

3. The method according to any one of claims 1 to 2, wherein said thickening agent is selected from a gelling agent, such as gellan gum, and/or wherein said non-toxic solubilizer and/or emulsifying agent is selected from a tenside.

4. The method according to any one of claims 1 to 3, wherein said olive or acebuche parts are selected from leaves, branches, twigs, root bark, stem bark, fruit or oil production residues or combinations thereof.

5. The method according to any one of claims 1 to 4, wherein said active agent comprises iridoids or phenols, and at least, one organic compound selected from the group of oleuropein, oleacein, oleocanthal, tyrosol, and hydroxytyrosol.

6. An antibacterial or antifungal composition comprising at least one organic compound selected from oleacein, hydroxytyrosol and oleocanthal.

7. The composition according to claim 6 for use in the prevention or treatment of a bacterial or fungal infection in mammals, preferably for use against infection by antibiotic resistant or multi-resistant bacteria.

8. An antibiotically or antifungally active sterilized pulverized olive or acebuche, obtained from steps a, b), c), and d) of the method according to claim 1.

9. The antibiotically or antifungally active sterilized pulverized acebuche according to claim 8, wherein said pulverized olive or acebuche is a powder and consists of more than 95% by weight of dry biomass.

10. An olive or acebuche composition, produced according to any one of claims 1 to 5, optionally comprising pharmaceutically or cosmetically acceptable carriers, diluents or excipients, and wherein preferably said composition is a gel, preferably a homogenous colloidal gel, and wherein preferably said olive or acebuche suspension is an aqueous suspension.

11. The olive or acebuche composition according to claim 10, wherein said composition is a suspension, and wherein the amount of the non-toxic solubilizer is less than 10%, preferably less than 5%, and more preferably less than 3% of the overall volume of the olive or acebuche suspension.

12. The composition according to any one of claims 10 to 11 in the form of a salve, a lotion, a cream, spray, a gel, a liquid, drops, a capsule or a suppository.

13. The sterilized pulverized olive or acebuche according to claim 8 or 9, the olive or acebuche composition according to any one of claims 10 to 12 for use in the prevention or treatment of a bacterial or fungal infection in mammals, preferably infection by antibiotic resistant or multi-resistant bacteria, wherein preferably a) said bacteria are selected from *Escherichia coli, Staphylococcus aureus, Pseudomonas aeruginosa, Klebsiella pneumoniae, Mycobacterium tuberculosis, Enterococcus hirae, Enterococcus faecalis, Enterobacter, Serratia, Proteus, Providencia, Morganella, Enterococcus faecium, Heliocobacter pylori, Campylobacter, Salmonella, Neisseria gonorrhoeae, Streptococcus pneumoniae, Haemophilus influenza, Shigella, Acinetobacter baumannii,* and resistant and multi-resistant strains thereof, and, wherein b) said fungi are selected from *Candida albicans* and *Aspergillus brasiliensis.*

14. Use of the sterilized pulverized olive or acebuche according to claim 8 or 9, or the olive or acebuche composition according to any one of claims 10 to 12 as a functional food additive, in particular an animal feed additive.

15. Use of the sterilized pulverized olive or acebuche according to claim 8 or 9, the olive or acebuche composition according to any one of claims 10 to 12 in cosmetics.
